# NEW EUROPEAN PATENT SPECIFICATION

(11) **EP 0 365 301 B2**
(45) Date of publication and mention of the opposition decision: **25.09.2002**
(45) Mention of the grant of the patent: 31.08.1994
(21) Application number: 89310703.7
(22) Date of filing: 18.10.1989
(51) Int. Cl.: A61B 6/03

(54) **Imaging apparatuses and methods**
Verfahren und Gerät für Bilddarstellungen
Appareils et procédés de représentation d'images

(30) Priority: 20.10.1988 US 260403
(43) Date of publication of application: 25.04.1990
(73) Proprietor: PICKER INTERNATIONAL, INC., Highland Heights Ohio 44143 (US)
(72) Inventor: Heuscher, Dominic J., Aurora Ohio 44202 (US); Mattson, Rodney A., Mentor Ohio 44060 (US); Brunnett, Carl J, Willoughby Hills, Ohio 44094 (US)
(74) Representative: McGowan, Nigel George

(56) References cited:
- EP-A- 0 112 475
- EP-A- 0 230 155
- EP-A- 0 245 147
- DE-A- 2 726 635
- DE-A- 2 932 182
- US-A- 3 973 128
- US-A- 4 206 359
- US-A- 4 298 800
- US-A- 4 383 327
- US-A- 4 442 489

## Description

This invention relates to imaging apparatuses and methods. More particularly the invention relates to medical diagnostic imaging apparatuses and methods, especially computed tomography (CT) apparatuses and methods. The invention finds application in conjunction with volumetric medical diagnostic imaging and will be described with particular reference thereto. However, it is to be appreciated that the invention also relates to single slice imaging, quality control examinations, and the like.

Heretofore, CT apparatus i.e. scanners have included a plurality of discrete radiation detectors arranged in a ring, or a section of a ring that is rotatable, around a patient examination region. Each discrete detector included a scintillation crystal which received radiation traversing a selected lice of a patient in the examination region and converted the radiation energy into light. A solid state photodiode or vacuum photomultiplier tube converted the light emitted by the scintillation crystal into electrical signals indicative of the intensity of emitted light, hence, the intensity of received radiation. By providing two adjacent rings of photodiodes or photomultiplier tubes back-to-back, two slices have been collected concurrently.

The use of discrete radiation detectors including photodiodes or photomultiplier tubes, has several drawbacks. Firstly, installation is labor-intensive and expensive. Further, the prior art scintillation crystal/photodiodes or photomultiplier tube detectors tend to be relatively bulky which limits the number which may be disposed in a ring around the examination region. This number may also be limited by the sampling frequency and data processing capacity of the scanner.

In such scanners, thick slices, created when the entire face of each discrete detector received radiation, suffered from visible partial volume effects, and narrow slices were created by screening part of each detector from receiving radiation. Further, the flux limitations of the photomultiplier tubes in such detectors required longer narrow slice scan times which lowered patient throughput.

It is an object of the present invention to provide an imaging apparatus and method wherein the above problems are overcome.

According to a first aspect of the present invention there is provided an imaging apparatus as defined in claim 1.

According to a second aspect of the present invention there is provided a method of imaging as defined in claim

One advantage of the present invention resides in the generation of volumetric data for a volume that may be defined by the fan beam projection of an x-ray source moving in a circular path as the position of a patent table is incremented. Alternatively, the x-ray source may move continuously in a circular path as the table also moves continuously. This results in the source following a helical path around the patient relative to the patient. In such arrangements the x-ray flux utilization is increased and the total examination period for a given volume is significantly reduced.

Another advantage of the present invention resides in its flexibility and imaging versatility. Multiple detectors can be grouped to maintain high photo efficiency while reducing the amount of data that has to be collected and processed. This can be done with a minimal reduction in resolution. If detectors are grouped in the axial direction, partial volume artifacts are significantly reduced over current scans of the same slice thickness.

Other advantages include being able to achieve multiple thin slices of more than one thickness. Because these slices are adjacent or nearly adjacent to each other, the off-plane effects are minimized.

EP-A-0112475 discloses an imaging apparatus comprising: means defining an examination region; radiation source means for rotating a beam of radiation about the examination region; means for receiving radiation that has traversed the examination region and producing electrical signals indicative of the received radiation, said means for receiving including a plurality of segmented arrays of radiation sensitive cells; and image reconstruction means for reconstructing the electrical signals into an image representation.

One imaging apparatus and method in accordance with the present invention will now be described, by way of example, with reference to the accompanying drawings in which:
Figure 1 is a diagrammatic illustration of the imaging apparatus;
Figure 2 illustrates the operation of a collimator of the apparatus;
Figure 3 is a diagrammatic illustration of a radiation detection means of the apparatus; and
Figure 4 is a diagrammatic illustration of an alternative radiation detetion means.

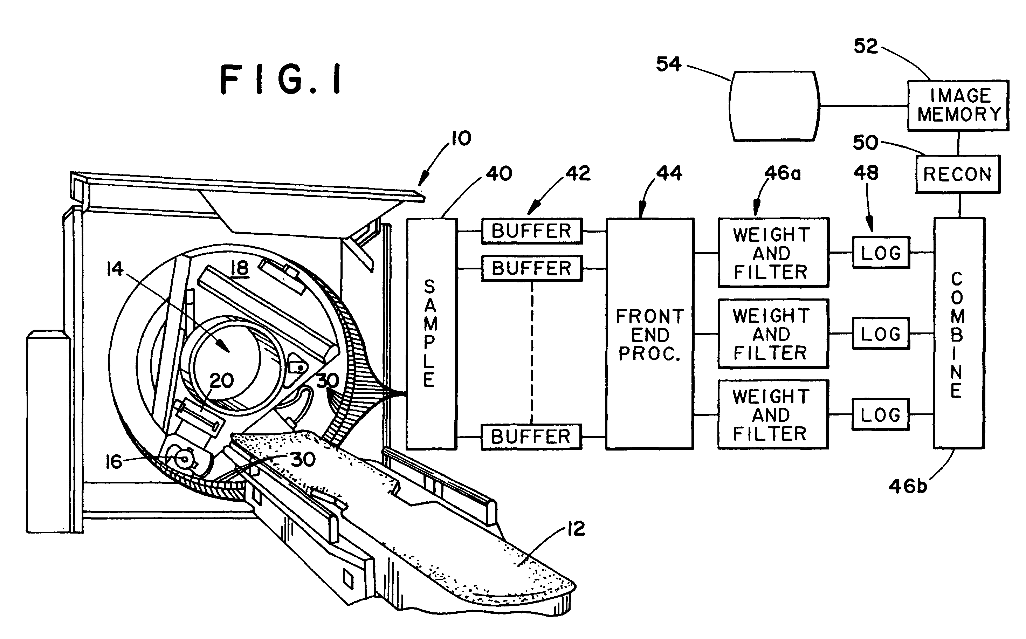

Referring to Figure 1, a CT scanner 10 selectively images cross sectional slices of a region of a patient supported on a stationary patient couch 12 within a scan circle or patient aperture 14. In some applications, the patient couch is incremented to take a plurality of parallel slices. In applications, the couch moves continuously such that the patient is scanned along a helical path. An x-ray tube 16 for emitting a fan-shaped beam of radiation toward and spanning the scan circle 14 is mounted to a rotatable gantry 18. Alternatively, a multi spot x-ray tube may be utilized to increase the thickness of the fan beam or generate plural parallel beams.

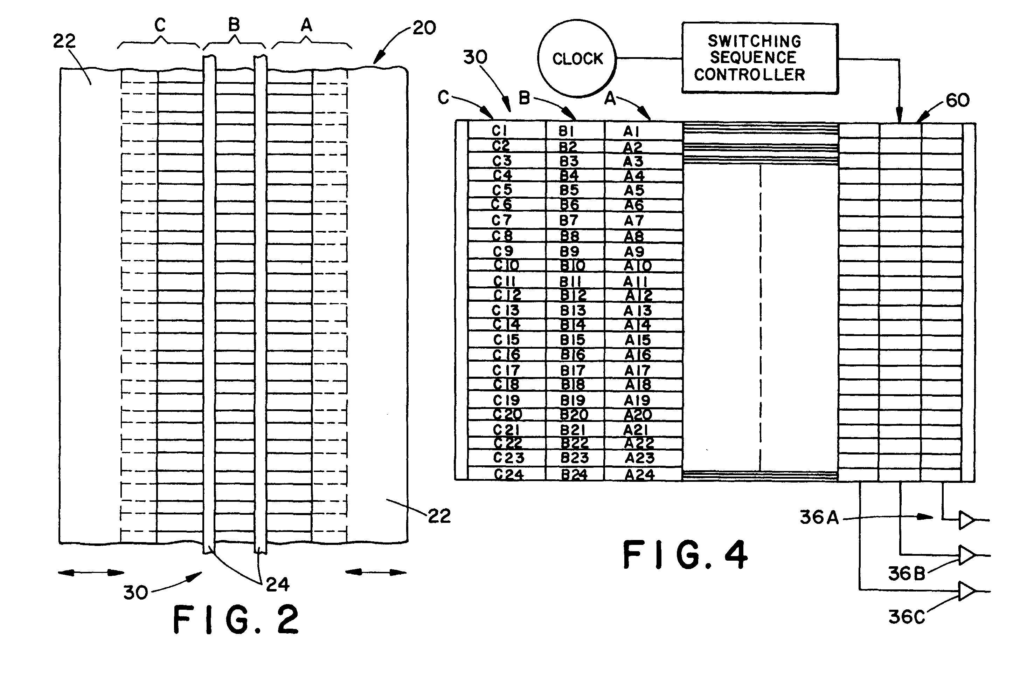

Referring also to Figure 2, a collimator 20 defines the dimensions of the x-ray beam(s), particularly its width to select the thicknesses of an individual slice or group of slices which are imaged. An outer continuously adjustable collimator part 22 sets the overall width of the x-ray beam, i.e. the width of the outer slices. If the outer collimator part 22 is closed sufficiently, the outer slices may be eliminated and the center or inner slice(s) can be narrowed. If the halves of the outer collimator part 22 are moved independently, one of the outer slices may be eliminated and the other adjusted to a selected width, e.g. the width of the inner slice. An inner collimator part 24 selectively narrows the center or inner slice. In the preferred embodiment, the inner collimator part 24 has a fixed profile which is selectively moved into and out of the radiation beam. Optionally, the fixed collimator segments may have selectable profiles such that the center slice collimation is adjustable. Other mechanical slice thickness adjustment or selection devices for the inner and outer slices may, of course, be utilized.

A plurality of segmented detector array modules 30 receive radiation which has traversed the scan circle and provide output signals indicative of the intensity of received radiation. In the preferred embodiment, 128 detector modules each include three columns of 24 photodiodes to define three rings of 2,880 photodiodes per ring around the scan circle.

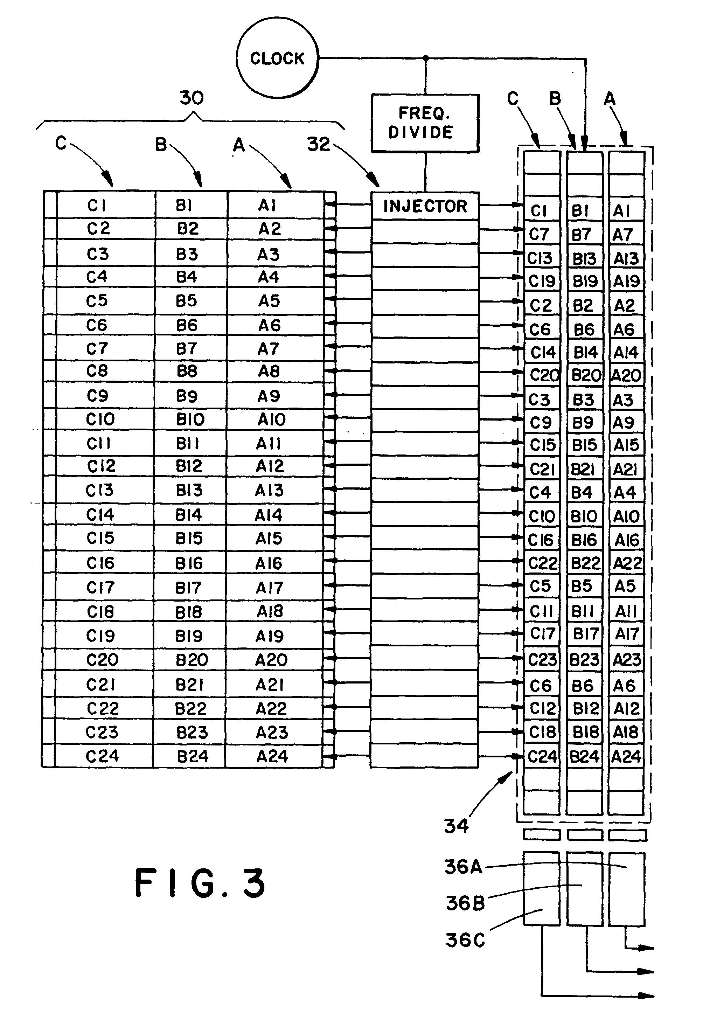

Referring to Figure 3, each detector module includes three columns A, B, C of 24 x-ray sensitive cells each. The cells of column A are labelled A1-A24, etc. Of course, other numbers of columns, such as five or more, may be provided and other numbers of radiation sensitive cells may be provided within each column without departing from the present invention. The center column B preferably is narrower than the side columns A and C. This enables the center column to have a higher resolution or define a narrower slice. By selectively adjusting the collimator 20, the amount of radiation impinging on the outer columns A and C and the amount impinging on the inner column B are selectively adjustable. This enables the slice defined by the outer columns to be the same as the center column, wider, or narrower. Within each column, there are a plurality of different height cells. In the preferred embodiment, taller cells A1, A3, A5,...A23, B1, B3,...B23, C1, C3,...C23 alternate with shorter cells A2, A4, A6...A24, B2, B4,...B24, C2, C4...C24.

In the preferred embodiment, the radiation sensitive cells of the modules 30 are photodiodes. An array of charge injectors 32 periodically, at the sampling interval, recharges each photodiode of the array to a preselected charge level. As radiation impinges on the photodiodes, the charge is dissipated in proportion to the intensity of radiation and the duration of exposure. At the end of each sampling interval when the injectors 32 recharge each photodiode, the injectors provide a like amount of charge to corresponding cells of a charge coupled device (CCD) array 34. The CCD array 34 has three columns of cells which in Figure 3 are labelled with the same designation as the corresponding photodiode of the photodiode array 30. The cells of the CCD array may be arranged in a different order than that of the corresponding diodes of the photodiode array to accommodate the order of data anticipated by downstream processing equipment. In this manner, the CCD array is loaded with charge values indicative of the amount of charge necessary to recharge each photodiode to the preselected charge, i.e. the amount of charge dissipated. Optionally, an indication of the charge remaining on each photodiode may be transferred to the CCD array.

After each sampling, the charge values in the cells of each column, A, B, and C of the CCD array are shifted rapidly to charge amplifiers 36a, 36b, and 36c, respectively. In the 3 x 24 cell array, each charge value is shifted in a time equal to 1/24th or less of the sampling interval. In this manner, the data from one sampling is converted to serial video signal data and the CCD array is ready to receive new data before the next sampling. If the three columns of cells are read serially rather than in parallel, the data is clocked out of each column three times as fast, such that all three columns are emptied before the next sampling.

Referring particularly to Figure 1, electrical output signals from amplifiers as 36a, 36b, and 36c indicative of the amount of radiation received by the photodiodes of each ring are sampled by a sampling means 40. Buffer memories 42 store the sampled data until a front end processor means 44 digitizes the signals and performs digital signal processing operations, as are known in the art. In the illustrated embodiment, each buffer receives data serially from one of the rings. A combining means 46a,46b includes an intra-ring weighting and filtering means 46a for selectively weighting or filtering the data from the different sized cells of each ring. Algorithm means 48 converts the data into its logarithmic values. In the illustrated embodiment, three logarithmic circuits each process the data from one ring.

The combining means 46a,46b further includes an axial combining means 46b for combining the data from the rings. In response to the operator selecting an imaging mode, the combining means selects a corresponding combination algorithm. The selected or combined data is reconstructed into an image representation by an image reconstruction means 50 such as a convolution and filtered back projection algorithm. The image representation may be stored in one or more image memories 52 for selective display on one or more video monitors 54. Alternately, the image representatons may be stored in computer memory, stored on tape, subject to further processing, or the like.

The operator can select various imaging modes including single and multiple slice modes with different resolutions. In the various modes, the outputs of the rings are combined in various manners, both axially and transversely or processed independently. For example, in one single slice mode, the outputs of each set of corresponding A, B, and C cells are combined. More specifically, the outer collimator part 22 adjusts the outer slice width and the inner collimator part 24 sets the width of the center slice. The three rings of cells receive the radiation and produce output signals which could be used to produce three slices. Unless the center and outside columns of cells are exposed to the same amount of radiation, the three slices have different resolutions. The corresponding cells of the three rings are weighted to accommodate the different resolutions, summed, averaged, or the like. In an arrangement in which the center cells are three millimeters wide and the outer ones, four millimeters wide, this single slice technique is applicable to single slices with a width of about three to ten millimeters.

In another single slice mode, only the center column of radiation cells is utilized. The adjustable outer collimator part 22 or inner collimator part 24 may be utilized to reduce the width of the single slice below the width of the center column of cells.

In a multiple slice mode, three slices are produced. That is, the data from each of the three rings of cells is processed separately. The center slice has the width of the center column of cells or the width defined by the inner collimator part 24. The width of the outer slices is separately adjusted by the outer collimator part 22. For many applications, it is advantageous to have all three slices the same width or resolution. In another multiple slice mode, two overlapping slices are produced. Data from corresponding cells of the center column and one column are combined to produce one set of data. Data from corresponding cells of the center column and the other side columm are also combined to produce a second set of data. The two sets of data, which represent overlapping slices, are processed separately. Again, the width of the slices may be adjusted with the collimator 20.

As yet another alternative, transverse combinations of radiation sensitive cells can be selected. In a spatially weighted view averaging mode, adjacent different sized cells within the same ring A, B, or C are combined or weighted. This effectively averages the views from which the resultant image is reconstructed. In a filtering mode, the data from adjacent cells within each ring is time averaged or weighted. In a view averaging mode, the outputs of adjacent cells within each ring are summed or averaged without filtering or weighting. As yet another alternative, the views that are summed or averaged may be the time filtered or spatially weighted views described above. Various other imaging modes or combinations of these imaging modes may, of course, be selected. The logarithm may be taken earlier or later in the processing stream. Preferably, the logarithm is taken before an axial combination to reduce partial volume effects.

Referring to Figure 4, the photodiode array 30 could alternatively be interconnected with an array of field effect transistor (FET) switches 60, rather than the CCD array as described above. The FETs are each connected with a respective one of the photodiodes for conveying an indication of the conductivity of the photodiode to one of the output amplifiers 36a, 36b and 36c. Preferably, an instantaneous voltage across a photodiode and resistor combination is measured. Optionally, other means besides a FET array or a CCD array may be provided for converting concurrently collected data into serial data signals.

## Claims

1. An imaging apparatus comprising: means (18) defining an examination region (14) for accommodating a subject to be imaged therein; radiation source means (16) for rotating a beam of radiation about the examination region (14); means (30, 32, 34 or 30, 60) for receiving radiation that has traversed the examination region (14) including radiation that has passed through a region of interest of the subject and producing electrical signals indicative of the received radiation, said means (30, 32, 34 or 30, 60) for receiving including a plurality of segmented arrays (30) of radiation sensitive cells (A1....A24, B1....B24, C1....C24); and image reconstruction means (50) for reconstructing the electrical signals into an image representation of the region of interest, **characterised in that** each segmented array (30) comprises a two dimensional regular grid of radiation sensitive cells (A1....A24, B1....B24, C1....C24) on a common substrate, the grid having a plurality of columns (A, B, C) of radiation sensitive cells (A1....A24, B1....B24, C1....C24) which extend along a circumference of the examination region (14) and a plurality of rows (1....24) of radiation sensitive cells (A1....A24, B1....B24, C1....C24) which extend in a direction lengthwise of the examination region (14), each segmented array (30) including cells (A1....A24, B1....B24, C1....C24) of different sizes, the cells (A1....A24, B1....B24, C1....C24) of different sizes in each segmented array (30) receiving radiation that has passed through the region of interest of the subject.

2. An apparatus according to Claim 1 wherein said means for receiving (30, 32, 34 or 30, 60) includes serialising means (32, 34 or 60) such that said electrical signals comprise at least one output signal of serial data values.

3. An apparatus according to Claim 2 wherein the serialising means (32, 34 or 60) includes: a charge coupled device array (34); and a charge injector means (32) for transferring to the charge coupled device array (34) charge values indicative of radiation received by the cells (A1....A24, B1....B24, C1....C24) of the segmented arrays (30), the charge coupled device array (34) being clocked to produce the or each output signal.

4. An apparatus according to Claim 2 wherein the serialising means (32, 34 or 60) includes an array (60) of field effect transistors on a common substrate.

5. An apparatus according to any one of the preceding claims further including: a sampling means (40) for sampling the electrical signals from each cell (A1....A24, B1....B24, C1....C24); and combining means (46 a, b) for selectively combining the sampled electrical signals.

6. An apparatus according to Claim 5 further including: logarithm means (48) for determining the logarithm of the sampled electrical signals, the logarithm means (48) being operatively connected with the combining means (46 a, b).

7. An apparatus according to Claim 5 or Claim 6 wherein the combining means (46 a, b) includes a weighting and filtering means (46a) for selectively spatially weighting and temporally filtering the electrical signals.

8. An apparatus according to Claim 1 further including a plurality of memory means (42), each memory means (42) storing electrical signals from a preselected subset of cells (A1....A24, B1....B24, C1....C24) of each segmented array (30), and combining means (46 a, b) for selectively combining stored electrical signals from the memory means (42), the combining means (46 a, b) being operatively connected with the image reconstruction means (50) for supplying the combined electrical signals thereto.

9. An apparatus according to Claim 1 or 8 further including: a charge coupled device array (34) comprising a plurality of columns (A, B, C) of charge coupled device cells (A1....A24, B1....B24, C1....C24); injector means (32) for transferring to the charge coupled device array (34) charge values indicative of radiation received by the cells (A1....A24, B1....B24, C1....C24) of the segmented arrays (30); a plurality of amplifiers (36 a, b, c), one connected with each column (A, B, C) of charge coupled device cells (A1....A24, B1....B24, C1....C24); and clock means for causing each amplifier (36 a, b, c) to amplify in series outputs from the cells (A1....A24, B1....B24, C1....C24) in its associated column (A, B, C) of charge coupled device cells (A1....A24, B1....B24, C1....C24).

10. An apparatus according to Claim 1 or 8 further including an array (60) of field effect transistors for connecting the radiation sensitive cells (A1....A24, B1....B24, C1....C24) to output amplifiers (36 a, b, c).

11. An apparatus according to any one of the preceding claims further including collimator means (20) for selectively screening a portion of the segmented arrays (30) of radiation sensitive cells (A1....A24, B1....B24, C1....C24).

12. An apparatus according to Claim 1 wherein the plurality of segmented arrays (30) form at least three rings of radiation sensitive cells (A1....A24, B1....B24, C1....C24) which encircle the examination region (14), each ring having more than 2,000 radiation sensitive cells (A1....A24, B1....B24, C1....C24) therein.

13. An apparatus according to any one of the preceding claims wherein the radiation sensitive cells (A1....A24, B1....B24, C1....C24) comprise photodiodes.

14. A method of imaging comprising: rotating a fan beam of radiation about an examination region (14) and thereby around a subject to be imaged disposed therein; detecting radiation which has traversed the examination region (14) including radiation that has passed through a region of interest of the subject using detection means (30) comprising a plurality of segmented arrays (30) of radiation sensitive cells (A1....A24, B1....B24, C1....C24); reading radiation absorption data from the detection means (30); and reconstructing an image representation of the region of interest from the read data, **characterised in that** each segmented array (30) used in said method comprises a two dimensional regular grid of radiation sensitive cells (A1....A24, B1....B24, C1....C24) on a common substrate, the grid having a plurality of columns (A, B, C) of radiation sensitive cells (A1....A24, B1....B24, C1....C24) which extend along a circumference of the examination region (14) and a plurality of rows (1....24) of radiation sensitive cells (A1....A24, B1....B24, C1....C24) which extend in a direction lengthwise of the examination region (14), each segmented array (30) including cells (A1....A24, B1....B24, C1....C24) of different sizes, the cells (A1....A24, B1....B24, C1....C24) of different sizes in each segmented array (30) receiving radiation that has passed through the region of interest of the subject.

## Patentansprüche

1. Bildgebendes Gerät mit: einem Mittel (18) zum Festlegen eines Untersuchungsbereichs (14), um darin ein abzubildendes Subjekt aufzunehmen; einem Strahlungsquellenmittel (16) zum Drehen eines Strahlungsbündels um den Untersuchungsbereich (14); einem Mittel (30, 32, 34 oder 30, 60) zum Empfangen von Strahlung, die den Untersuchungsbereich (14) durchquert hat, einschließlich Strahlung, die einen interessierenden Bereich des Subjektes durchlaufen hat, und Erzeugen elektrischer Signale, die für die empfangene Strahlung bezeichnend sind, wobei das Mittel (30, 32, 34 oder 30, 60) zum Empfangen eine Vielzahl von segmentierten Feldern (30) aus strahlungsempfindlichen Zellen (A1...A24, B1...B24, C1...C24) umfasst; und einem Bildrekonstruktionsmittel (50) zum Rekonstruieren der elektrischen Signale zu einer Bilddarstellung des interessierenden Bereiches, **dadurch gekennzeichnet, dass** jedes segmentierte Feld (30) ein zweidimensionales reguläres Gitter aus strahlungsempfindlichen Zellen (A1...A24, B1...B24, C1...C24) auf einem gemeinsamen Substrat umfasst, wobei das Gitter eine Vielzahl von Spalten (A, B, C) aus strahlungsempfindlichen Zellen (A1...A24, B1...B24, C1...C24) aufweist, die sich längs eines Umfangs des Untersuchungsbereiches (14) erstrecken, und eine Vielzahl von Reihen (1... 24) aus strahlungsempfindlichen Zellen (A1...A24, B1...B24, C1...C24), die sich in einer längs des Untersuchungsbereiches (14) verlaufenden Richtung erstrecken, wobei jedes segmentierte Feld (30) Zellen (A1...A24, B1...B24, C1...C24) unterschiedlicher Größe enthält, wobei die Zellen (A1...A24, B1...B24, C1...C24) unterschiedlicher Größe in jedem segmentierten Feld (30) Strahlung empfangen, die den interessierenden Bereich des Subjektes durchlaufen hat.

2. Gerät nach Anspruch 1, worin das Mittel zum Empfangen (30, 33, 34 oder 30, 60) ein Serialisierungsmittel (32, 34 oder 60) umfasst, sodass die elektrischen Signale zumindest ein Ausgangssignal aus seriellen Datenwerten umfassen.

3. Gerät nach Anspruch 2, worin das Serialisierungsmittel (32, 34 oder 60) umfasst: ein CCD-Feld (34) (CCD: charge-coupled device; ladungsgekoppelte Anordnung) und ein Ladungsinjektormittel (32) zum Übertragen von Ladungswerten, die für die Strahlung bezeichnend sind, die von den Zellen (A1...A24, B1...B24, C1...C24) der segmentierten Felder (30) empfangen worden sind, zu dem CCD-Feld (34), wobei das CCD-Feld (34) getaktet wird, um das oder jedes Ausgangssignal zu erzeugen.

4. Gerät nach Anspruch 2, worin das Serialisierungsmittel (32, 34 oder 60) ein Feld (60) aus Feldeffekttransistoren auf einem gemeinsamen Substrat umfasst.

5. Gerät nach einem der vorhergehenden Ansprüche, das weiterhin enthält: ein Abtastmittel (40) zum Abtasten der aus jeder Zelle (A1...A24, B1...B24, C1...C24) stammenden elektrischen Signale und ein Verknüpfungsmittel (46a, b) zum selektiven Kombinieren der abgetasteten elektrischen Signale.

6. Gerät nach Anspruch 5, das weiterhin enthält: ein Logarithmusmittel (48) zum Bestimmen des Logarithmus der abgetasteten elektrischen Signale, wobei das Logarithmusmittel (48) wirksam mit dem Verknüpfungsmittel (46a, b) verbunden ist.

7. Gerät nach Anspruch 5 oder 6, worin das Verknüpfungsmittel (46a, b) ein Gewichtungs- und Filtermittel (46a) zum selektiven räumlichen Gewichten und zeitlichen Filtern der elektrischen Signale enthält.

8. Gerät nach Anspruch 1, das weiterhin eine Vielzahl von Speichermitteln (42) enthält, wobei jedes Speichermittel (42) aus einer zuvor selektierten Teilmenge von Zellen (A1...A24, B1...B24, C1...C24) jedes segmentierten Feldes (30) stammende elektrische Signale speichert, und ein Verknüpfungsmittel (46a, b) zum selektiven Kombinieren gespeicherter, aus dem Speichermittel (42) stammender elektrischer Signale, wobei das Verknüpfungsmittel (46a, b) wirksam mit dem Bildrekonstruktionsmittel (50) verbunden ist, um ihnen die kombinierten elektrischen Signale zuzuführen.

9. Gerät nach Anspruch 1 oder 8, das weiterhin enthält: ein CCD-Feld (34) mit einer Vielzahl von Spalten (A, B, C) von CCD-Zellen (A1...A24, B1...B24, C1...C24); ein Injektormittel (32) zum Übertragen von Ladungswerten, die bezeichnend für Strahlung sind, die von den Zellen (A1...A24, B1...B24, C1...C24) der segmentierten Felder (30) empfangen worden ist, zu dem CCD-Feld (34); eine Vielzahl von Verstärkern (36a, b, c), wobei einer mit jeder Spalte (A, B, C) von CCD-Zellen (A1...A24, B1...B24, C1...C24) verbunden ist, und einem Taktmittel, um jeden Verstärker (36a, b, c) zu veranlassen, Ausgangssignale aus den Zellen (A1...A24, B1...B24, C1...C24) in seiner zugehörigen Spalte (A, B, C) von CCD-Zellen (A1...A24, B1...B24, C1...C24) in Serie zu verstärken.

10. Gerät nach Anspruch 1 oder 8, das weiterhin ein Feld (60) aus Feldeffekttransistoren zum Verbinden der strahlungsempfindlichen Zellen (A1...A24, B1...B24, C1...C24) mit Ausgangsverstärkern (36a, b, c) enthält.

11. Gerät nach einem der vorhergehenden Ansprüche, das weiterhin ein Kollimatormittel (20) zum selektiven Abschirmen eines Teils der segmentierten Felder (30) aus strahlungsempfindlichen Zellen (A1...A24, B1...B24, C1...C24) enthält.

12. Gerät nach Anspruch 1, worin die Vielzahl von segmentierten Feldern (30) zumindest drei Ringe aus strahlungsempfindlichen Zellen (A1...A24, B1...B24, C1...C24) bilden, die den Untersuchungsbereich (14) umgeben, wobei jeder Ring darin mehr als 2000 strahlungsempfindliche Zellen (A1...A24, B1...B24, C1...C24) aufweist.

13. Gerät nach einem der vorhergehenden Ansprüche, worin die strahlungsempfindlichen Zellen (A1...A24, B1...B24, C1...C24) Photodioden umfassen.

14. Verfahren des Bildgebens mit den Schritten: Drehen eines fächerförmigen Strahlungsbündels um einen Untersuchungsbereich (14) und dadurch um ein darin angeordnetes, abzubildendes Subjekt; Detektieren von Strahlung, die den Untersuchungsbereich (14) durchquert hat, einschließlich Strahlung, die einen interessierenden Bereich des Subjektes durchlaufen hat, unter Verwendung von Detektionsmitteln (30), die eine Vielzahl von segmentierten Feldern (30) aus strahlungsempfindlichen Zellen (A1...A24, B1...B24, C1...C24) umfassen; Auslesen von Strahlungsabsoptionsdaten aus den Detektionsmitteln (30) und Rekonstruieren einer Bilddarstellung des interessierenden Bereiches aus den ausgelesenen Daten, **dadurch gekennzeichnet, dass** jedes in dem genannten Verfahren verwendete segmentierte Feld (30) ein zweidimensionales reguläres Gitter aus strahlungsempfindlichen Zellen (A1...A24, B1...B24, C1...C24) auf einem gemeinsamen Substrat umfasst, wobei das Gitter eine Vielzahl von Spalten (A, B, C) aus strahlungsempfindlichen Zellen (A1...A24, B1...B24, C1...C24) aufweist, die sich längs eines Umfangs des Untersuchungsbereiches (14) erstrecken, und eine Vielzahl von Reihen (1... 24) aus strahlungsempfindlichen Zellen (A1...A24, B1...B24, C1...C24), die sich in einer längs des Untersuchungsbereiches (14) verlaufenden Richtung erstrecken, wobei jedes segmentierte Feld (30) Zellen (A1...A24, B1...B24, C1...C24) unterschiedlicher Größe enthält, wobei die Zellen (A1...A24, B1...B24, C1...C24) unterschiedlicher Größe in jedem segmentierten Feld (30) Strahlung empfangen, die den interessierenden Bereich des Subjektes durchlaufen hat.

## Revendications

1. Appareil de représentation d'images comprenant: des moyens (18) définissant une région d'examen (14) pour recevoir un objet à y former ; des moyens de source de rayonnement (16) pour faire tourner un faisceau de rayonnement autour de la région d'examen (14); des moyens (30, 32, 34 ou 30, 60) pour recevoir le rayonnement qui a traversé la région d'examen (14) incorporant le rayonnement qui a traversé une région d'intérêt de l'objet et pour produire des signaux électriques indicatifs du rayonnement reçu, lesdits moyens (30, 32, 34 ou 30, 60) pour recevoir le rayonnement comprenant une pluralité de groupements segmentés (30) de cellules sensibles au rayonnement (A1...A24, B1...B24, C1...C24); et des moyens de reconstruction d'images (50) pour convertir les signaux électriques en une représentation sous forme d'image de la région d'intérêt, **caractérisé en ce que** chaque groupement segmenté (30) comprend une grille bidimensionnelle régulière de cellules sensibles au rayonnement (A1...A24, B1...B24, C1...C24) disposées sur un substrat commun, la grille ayant une pluralité de colonnes (A, B, C) de cellules sensibles au rayonnement (A1...A24, B1...B24, C1...C24) qui s'étendent le long d'une circonférence de la région d'examen (14) et une pluralité de rangées (1...24) de cellules sensibles au rayonnement (A1...A24, B1...B24, C1...C24) qui s'étendent dans la direction de la longueur de la région d'examen (14), chaque groupement segmenté (30) incorporant des cellules (A1...A24, B1...B24, C1...C24) de tailles différentes, les cellules (A1...A24, B1...B24, C1...C24) de tailles différentes dans chaque groupement segmenté (30) recevant le rayonnement qui a traversé la région d'intérêt de l'objet.

2. Appareil selon la revendication 1, dans lequel lesdits moyens pour recevoir le rayonnement (30, 32, 34 ou 30, 60) comportent des moyens de sérialisation (32, 34 ou 60) tels que lesdits signaux électriques comprennent au moins un signal de sortie de valeurs de données en série.

3. Appareil selon la revendication 2, dans lequel les moyens de sérialisation (32, 34 ou 60) comprennent: un groupement de dispositifs à couplage de charge (34); et des moyens d'injection de charge (32) pour transférer au groupement de dispositifs à couplage de charge (34) des valeurs de charge indicatives du rayonnement reçu par les cellules (A1...A24, B1...B24, C1...C24) des groupements segmentés (30), le groupement de dispositifs à couplage de charge (34) recevant des signaux d'horloge pour produire le signal de sortie ou chaque signal de sortie.

4. Appareil selon la revendication 2, dans lequel les moyens de sérialisation (32, 34 ou 60) comprennent un groupement (60) de transistors à effet de champ disposés sur un substrat commun.

5. Appareil selon l'une quelconque des revendications précédentes, comprenant en outre: des moyens d'échantillonnage (40) pour échantillonner les signaux électriques issus de chaque cellule (A1...A24, B1...B24, C1...C24); et des moyens combinateurs (46a, b) pour combiner sélectivement les signaux électriques échantillonnés.

6. Appareil selon la revendication 5, comprenant en outre: des moyens de logarithme (48) pour déterminer le logarithme des signaux électriques échantillonnés, les moyens de logarithme (48) étant reliés fonctionnellement aux moyens combinateurs (46a,b).

7. Appareil selon la revendication 5 ou la revendication 6, dans lequel les moyens combinateurs (46a, b) comprennent des moyens de pondération et de filtrage (46a) pour pondérer d'une manière sélective spatialement et pour filtrer d'une manière sélective les signaux électriques.

8. Appareil selon la revendication 1, comportant encore une pluralité de moyens de mémoire (42), chaque moyen de mémoire (42) mémorisant des signaux électriques issus d'un sous-ensemble de cellules (A1...A24, B1...B24, C1...C24) de chaque groupement segmenté (30), et des moyens combinateurs (46a, b) pour combiner sélectivement des signaux électriques mémorisés issus des moyens de mémoire (42), les moyens combinateurs (46a, b) étant reliés fonctionnellement aux moyens de reconstruction d'images (50) pour fournir les signaux électriques combinés à ceux-ci.

9. Appareil selon la revendication 1 ou la revendication 8, comprenant en outre: un groupement de dispositifs à couplage de charge (34) comprenant une pluralité de colonnes (A, B,C) de cellules à dispositif à couplage de charge (A1...A24, B1...B24, C1...C24); des moyens d'injection (32) pour transférer au groupement de dispositifs à couplage de charge (34) des valeurs de charge indicatives du rayonnement reçu par les cellules (A1...A24, B1...B24, C1...C24) des groupements segmentés (30); une pluralité d'amplificateurs (36a, b, c), un connecté à chaque colonne (A, B, C) de cellules de dispositifs à couplage de charge (A1...A24, B1...B24, C1...C24); et des moyens d'horloge pour amener chaque amplificateur (36a, b, c) à amplifier en série les signaux de sortie des cellules (A1...A24, B1...B24, C1...C24) dans sa colonne associée (A, B, C) de cellules de dispositifs à couplage de charge (A1...A24, B1...B24, C1...C24).

10. Appareil selon la revendication 1 ou la revendication 8, comprenant en outre un groupement (60) de transistors à effet de champ pour connecter les cellules sensibles au rayonnement (A1...A24, B1...B24, C1...C24) aux amplificateurs de sortie (36a, b, c).

11. Appareil selon l'une quelconque des revendications précédentes, comprenant en outre des moyens collimateurs (20) pour masquer sélectivement une partie des groupements segmentés (30) de cellules sensibles au rayonnement (A1...A24, B1...B24, C1...C24).

12. Appareil selon la revendication 1, dans lequel la pluralité de groupements segmentés (30) forme au moins trois anneaux de cellules sensibles au rayonnement (A1...A24, B1...B24, C1...C24) qui encerclent la région d'examen (14), chaque anneau ayant plus de 2000 cellules sensibles au rayonnement (A1...A24, B1...B24, C1...C24) dans celui-ci.

13. Appareil selon l'une quelconque des revendications précédentes, dans lequel les cellules sensibles au rayonnement (A1...A24, B1...B24, C1...C24) sont des photodiodes.

14. Procédé de représentation d'images, comprenant les étapes consistant à: faire tourner un faisceau de rayonnement en éventail autour d'une région d'examen (14) et, par conséquent, autour d'un objet à visualiser disposé dans celle-ci; détecter le rayonnement qui a traversé la région d'examen (14) incorporant le rayonnement qui a traversé une région d'intérêt de l'objet en utilisant des moyens de détection (30) comprenant une pluralité de groupements segmentés (30) de cellules sensibles au rayonnement (A1...A24, B1...B24, C1...C24); lire les données d'absorption de rayonnement issues des moyens de détection (30); et reconstruire une représentation sous forme d'image de la région d'intérêt à partir des données lues, **caractérisé en ce que** chaque groupement segmenté (30) utilisé dans ledit procédé comprend une grille bidimensionnelle régulière de cellules sensibles au rayonnement (A1...A24, B1...B24, C1...C24) disposées sur un substrat commun, la grille ayant une pluralité de colonnes (A, B, C) de cellules sensibles au rayonnement (A1...A24, B1...B24, C1...C24) qui s'étendent le long d'une circonférence de la région d'examen (14) et une pluralité de rangées (1, ..., 24) de cellules sensibles au rayonnement (A1...A24, B1...B24, C1...C24) qui s'étendent dans la direction de la longueur de la région d'examen (14), chaque groupement segmenté (30) incorporant des cellules (A1...A24, B1...B24, C1...C24) de tailles différentes dans chaque groupement segmenté (30) recevant le rayonnement qui a traversé la région d'intérêt de l'objet.
